# EUROPÄISCHE PATENTSCHRIFT

(11) **EP 0 965 574 B1**
(45) Veröffentlichungstag und Bekanntmachung des Hinweises auf die Patenterteilung: **13.10.2004**
(21) Anmeldenummer: 99111460.4
(22) Anmeldetag: 12.06.1999
(51) Int. Cl.: B01J 31/22, C07B 53/00

(54) **Verfahren zur enantioselektiven Hydrierung**
Process for enantioselective hydrogenation
Procédé d'hydrogénation énantiosélective

(30) Priorität: 19.06.1998 DE 19827311; 19.12.1998 DE 19858866; 12.05.1999 DE 19921924
(43) Veröffentlichungstag der Anmeldung: 22.12.1999
(73) Patentinhaber: Degussa AG, 40474 Düsseldorf (DE)
(72) Erfinder: Knochel, Paul Prof., 35037 Marburg (DE); Almena Perea, Juan José Dr., 63450 Hanau (DE); Drauz, Karlheinz Prof., 63579 Freigericht (DE); Klement, Ingo Dr., 35415 Pohlheim-Garbenteich (DE)

(56) Entgegenhaltungen:
- US-A- 5 596 114
- JAHYO K ET AL: "Asymmetric Synthesis of A New Cylindrically Chiral and Air-Stable Ferrocenyldiphosphine and Its Application to Rhodium-Catalyzed Asymmetric Hydrogenation" TETRAHEDRON LETTERS,NL,ELSEVIER SCIENCE PUBLISHERS, AMSTERDAM, Bd. 39, Nr. 31, 30. Juli 1998 (1998-07-30), Seiten 5523-5526, XP004124106 ISSN: 0040-4039
- BURK M J ET AL: "EFFICIENT RHODIUM-CATALYZED HYDROGENATION OF ALDEHYDES AND KETONES" TETRAHEDRON LETTERS,NL,ELSEVIER SCIENCE PUBLISHERS, AMSTERDAM, Bd. 35, Nr. 28, 11. Juli 1994 (1994-07-11), Seiten 4963-4966, XP000465945 ISSN: 0040-4039
- SCHWINK L. ET AL: 'New C2-symmetrical ferrocenyl diamines as ligands ...' TETRAHEDRON: ASSYMETRY Bd. 9, 1998, Seiten 1143 - 1163

## Beschreibung

Die vorliegende Erfindung betrifft ein Verfahren zur homogenen katalytischen enantioselektiven Hydrierung von Verbindungen der allgemeinen Formel (I) 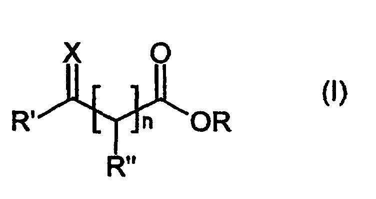 worin
n 0 oder 1 ist,
R = H, (C₁-C₁₈)-Alkyl, (C₆-C₁₈)-Aryl, (C₇-C₁₉)-Aralkyl, (C₁-C₈)-Alkyl- (C₆-C₁₈)-Aryl, (C₃-C₈)-Cycloalkyl, (C₁-C₈)-Alkyl- (C₃-C₈)-Cycloalkyl, (C₃-C₈)-Cycloalkyl- (C₁-C₈)-Alkyl,
   oder eine Carboxyschutzgruppe bedeutet,
R' = H, (C₁-C₁₈)-Alkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl- (C₆-C₁₈)-Aryl, (C₁-C₈)-Alkyl- (C₃-C₁₉)-Heteroaryl, (C₃-C₈)-Cycloalkyl, (C₁-C₈)-Alkyl- (C₃-C₈)-Cycloalkyl, (C₃-C₈)-Cycloalkyl-(C₁-C₈)-Alkyl, wobei die eben genannten Reste mit (C₁-C₈)-Acyl oder (C₁-C₈)-Alkyloxycarbonyl substituiert sein können, bedeutet, wobei (C₃-C₁₈)-Heteroarylrest ein fünf-, sechs- oder siebengliedriges aromatisches Ringsystem aus 3 bis 18 C-Atomen, welches Heteroatome ausgewählt aus Stickstoff, Sauerstoff oder Schwefel im Ring aufweist, bezeichnet,
X = O, CHR'', NR'', ist,
R'' = H, OH, R', (C₁-C₁₈)-Alkoxy, (C₂-C₁₈)-Alkoxyalkyl, (C₁-C₁₈)-Acyl, (C₁-C₁₈)-Acyloxy sein kann, wobei für verschiedene Positionen im Molekül R'' verschiedene Gestalt annehmen kann,
oder R und oder R' und R'' oder R'' und R sind über eine (C₁-C₄)-Brücke miteinander verbunden, welche einfach oder mehrfach mit linear oder verzweigten (C₁-C₈)-Alkyl, (C₁-C₈)-Acyl, (C₁-C₈)-Alkoxy, (C₂-C₈)-Alkoxyalkylresten substituiert sein und/oder im Ring Heteroatome ausgewählt aus N, O, P, S enthalten kann,
oder worin
n 1 ist,
R = H, (C₁-C₁₈)-Alkyl, (C₆-C₁₈)-Aryl, (C₇-C₁₉)-Aralkyl, (C₁-C₈)-Alkyl-(C₆-C₁₈)-Aryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
   oder eine Carboxyschutzgruppe bedeutet,
R' = H, (C₁-C₁₈)-Alkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl- (C₃-C₈)-Cycloalkyl, (C₃-C₈)-Cycloalkyl-(C₁-C₈)-Alkyl, wobei die eben genannten Reste mit (C₁-C₈)-Acyl oder (C₁-C₈)-Alkyloxycarbonyl substituiert sein können, bedeutet,
   wobei (C₃-C₁₈)-Heteroarylrest ein fünf-, sechs- oder siebengliedriges aromatisches Ringsystem aus 3 bis 18 C-Atomen, welches Heteroatome ausgewählt aus Stickstoff, Sauerstoff oder Schwefel im Ring aufweist, bezeichnet,
X = NR'' ist,
R'' = H, OH, R', (C₁-C₁₈)-Alkoxy, (C₂-C₁₈)-Alkoxyalkyl, (C₁-C₁₈)-Acyl, (C₁-C₁₈)-Acyloxy sein kann, wobei für verschiedene Positionen R'' verschiedene Gestalt annehmen kann,
oder R und R' oder R' und R'' oder R'' und R sind über eine (C₁-C₄)-Brücke miteinander verbunden, welche einfach oder mehrfach mit linear oder verzweigten (C₁-C₈)-Alkyl, (C₁-C₈)-Acyl, (C₁-C₈)-Alkoxy, (C₂-C₈)-Alkoxyalkylresten substituiert sein und/oder im Ring Heteroatome ausgewählt aus N, O, P, S enthalten kann, mit Hilfe von *C*₂-symmetrischen Ferrocenylkatalysatoren.

Die durch die erfindungsgemäße enantioselektive Hydrierung hergestellten Derivate können als chirale Intermediate in der organischen Synthese verwendet werden.

Die enantioselektive Einführung von stereogenen Zentren in organische Moleküle durch homogen katalysierte Hydrierung ist für spezielle Anwendungen im industriellen Maßstab etabliert. Die enantioselektiven Produkte sind wertvolle Ausgangssubstanzen zur Herstellung bioaktiver Wirkstoffe.

Der Einsatz von Bisphosphinkatalysatoren für die enantioselektive homogene katalytische Hydrierung für den eben genannten Zweck ist wohl bekannt (Burk et al., Tetrahedron 1994, 4399)

Knochel et al. (Chem. Eur. J. 1998, 4, 950-968), Hayashi et al. (J. Chem. Soc., Chem. Commun. 1989, 495-496) und Ikeda et al. (Tetrahedron Lett. 1996, 4545-4448) beschreiben Pd-Komplexe mit *C*₂-symmetrische Ferrocenyl-(bis-tertiärphosphin)-Liganden. Allerdings wurden diese Komplexe lediglich bei asymmetrischen Allylierungen eingesetzt. Deren Verwendung als Katalysatoren bei der enantioselektiven Hydrierung ist bislang nicht bekannt.

Yamamoto et al. (Bull. Chem. Soc. Jpn. 1980, 53, 1132-1137) berichteten über den Einsatz von nicht *C*₂-symmetrischen Ferrocenyl-(bis-tertiär-phosphin)-Liganden in der enantioselektiven homogenen katalytischen Hydrierung. Mit diesen Liganden erhält man jedoch nur sehr vereinzelt gute Enantiomerenüberschüsse.

Die DE 19827311.8 und Kang et al. (Tetrahedron Lett. 1998, 39, 5523-5526) offenbaren *C*₂-symmetrische Ferrocenylkomplexe als geeignete Katalysatoren für die enantioselektive Hydrierung von Acetamidozimtsäurederivate.

Aufgabe der vorliegenden Erfindung ist die Angabe eines Verfahrens zur homogenen katalytischen enantioselektiven Hydrierung weiterer ungesättigter Systeme mit Hilfe *C*₂-symmetrischer Ferrocenylkatalysatoren.

Unter Mehrfachbindungen werden im Rahmen der Erfindung Doppel-Bindungen zwischen einem Kohlenstoffatom und einem weiteren Kohlenstoffatom oder Sauerstoffatom oder Stickstoffatom verstanden.

Gelöst wird diese Aufgabe durch ein Verfahren gemäß Anspruch 1. Bevorzugte Ausführungsformen sind Gegenstand der von Anspruch 1 abhängigen Unteransprüche.

Dadurch, daß man zur homogenen katalytischen enantioselektiven Hydrierung von Verbindungen der allgemeinen Formel (I)

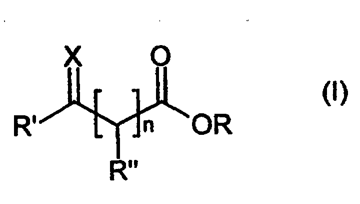

Katalysatoren der allgemeinen Formel (II) 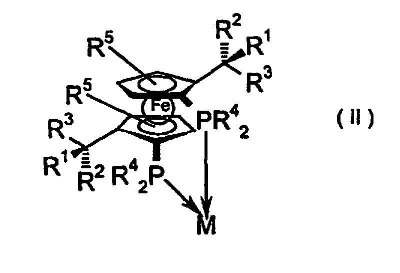 worin
R¹, R² unabhängig voneinander bedeuten H, O(C₁-C₈)-Acyl, N(C₁-C₈)-Alkyl₂, (C₁-C₈)-Alkyl
R³ bedeutet (C₆-C₁₈)-Aryl,
R⁴ bedeutet Phenyl,
R⁵ bedeutet H.
und M ein Metallatom oder -ion der Nebengruppe 7 oder 8, wie z. B. Co, Ni, Rh, Ru, Ir, Pd, Re oder Pt ist,
einsetzt, gelangt man in nichtvorhersehbarer Weise in guten bis sehr guten Ausbeuten und mit guten bis sehr guten Enantiomerenüberschüssen zu den gewünschten hydrierten Derivaten.

Das erfindungsgemäße Verfahren kann vorteilhafter Weise bei einer Temperatur zwischen 0°C und 150°C, vorzugsweise zwischen 20°C und 80°C, durchgeführt werden.

Der Wasserstoffdruck sollte während der Reaktion zwischen 10 kPa und 10000 kPa, vorzugsweise zwischen 50 kPa und 8000 kPa, liegen.

Als Lösungsmittel können alle dem Fachmann geläufigen Lösungsmittel eingesetzt werden, vorausgesetzt sie stören diese nicht im Hinblick auf Ausbeute und chirale Induktion. Bevorzugt sind Ether, wie THF, DME, MTBE, Alkohole, wie MeOH, EtOH, Propanol, Butanol, zu verwenden.

Die Bereitstellung der *C*₂-symmetrischen Katalysatoren kann entsprechend der DE 19827311.8 erfolgen.

Die Ferrocenylkatalysatoren zeigen bei der homogenen enantioselektiven katalytischen Hydrierung ausgezeichnete Werte, wie folgende Tabelle 1 belegt.

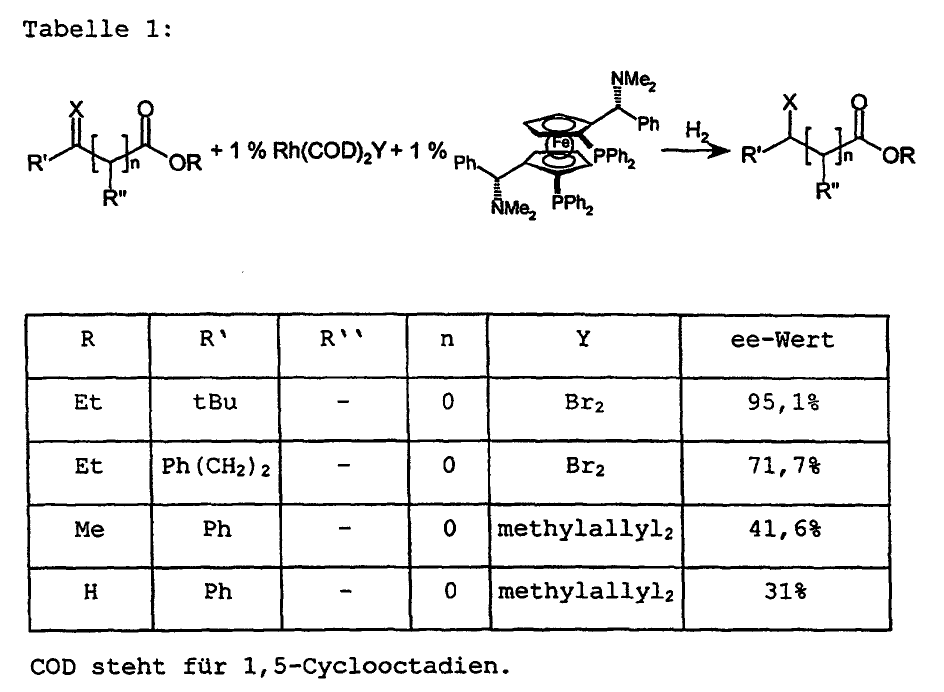

Die Katalysatorkonzentration ist mit 1% in den genannten Beispielen bereits sehr niedrig. Sie kann jedoch für technische Anwendung weiter erniedrigt werden. Dies beides ist für die Anwendung der erfindungsgemäßen Liganden im technischen Maßstab sehr von Vorteil, da die Kosten für die nach diesem Verfahren gewonnenen Produkte entsprechend niedriger ausfallen und damit ein höherer ökonomischer Nutzen als bei Verwendung von Ligandensystemen des Standes der Technik garantiert wird.

Nutzen als bei Verwendung von Ligandensystemen des Standes der Technik garantiert wird.

Der Rest R⁵ in den Komplexen kann u. a. zur Anbindung der erfindungsgemäßen Komplexe an eine polymere Matrix wie z. B. ein lineares PMMA, Polystyrol oder PEG sowie ein nichtlineares Dendrimer benutzt werden. Die Anbindung des Restes R⁵ an den Cyclopentadienylring des erfindungsgemäßen Komplexes ist bzgl. der freien Positionen und bzgl. des Ringes variabel. Mithin genügt die Anbindung des Polymers an einen Ring. Als Reste können alle dem Fachmann für diesen Zweck in Frage kommenden Reste verwandt werden. Eine geeignete Übersicht zur molekularen Vergrößerung von Komplexkatalysatoren bietet (Tetrahedron Asymmetry 1998, *9*, 691-696). Bevorzugt besteht der Rest R⁵ aus der Anordnung B-X-Z, wobei B ein Rest der Gruppe CR⁸₂, NR⁸, O, S, SiR⁸₂, X ein Spacer, wie z. B. 1,4*'*-Biphenyl, 1-, 2-Ethylen, 1-, 3-Propylen, PEG-(2-10) und Z eine funktionelle Gruppe, wie z. B. die O-, NH-, COO-, CONH, Ethenyl-, NHCONH-, OCONH- oder NHCOO-Funktion, welche an ein wie oben geschildertes Polymer gebunden ist. Alternativ können die Reste R⁵ der beiden Cyclopentadienylringe über eine α,ω,-(C₂-C₄)-Alkylenbrücke miteinander verbunden sein.

Das sich mit den *C*₂-symmetrischen Ferrocenylkomplexe neben den Acetamidozimtsäurederivaten auch andere ungesättigte Verbindungen in zum Teil recht hohen ee-Werten hydrieren lassen, war im Stand der Technik bisher nicht bekannt. Um so überraschender aber nicht minder vorteilhaft ist es, daß auch Verbindungen des Typs (I) enantioselektiv umgesetzt werden können.

Als linear oder verzweigte (C₁-C₁₈)-Alkyl sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl bis zu einem 18 C-Atome aufweisenden Rest samt aller seiner Bindungsisomeren. Der Rest (C₁-C₁₈)-Alkoxy entspricht dem Rest (C₁-C₁₈)-Alkyl mit der Maßgabe, daß dieser über ein Sauerstoffatom an das Molekül gebunden ist. Als (C₂-C₁₈)-Alkoxyalkyl sind Reste gemeint, bei denen die Alkylkette durch mindestens eine Sauerstoffunktion unterbrochen ist, wobein nicht zwei Sauerstoffatome miteinander verbunden sein können. Die Anzahl der Kohlenstoffatome gibt die Gesamtzahl der im Rest enthaltenen Kohlenstoffatome an.

Die eben beschriebenen Reste können einfach oder mehrfach mit Halogenen und/oder N-, O-, P-, S-atomhaltige Reste substituiert sein. Dies sind insbesondere Alkylreste der oben genannten Art, welche eines oder mehrere dieser Heteroatome in ihrer Kette aufweisen bzw. welche über eines dieser Heteroatome an das Molekül gebunden sind. Für die (C₁-C₈)-Alkylreste gilt das oben gesagte entsprechend für einen max. 8 C-Atome enthaltenden Alkylrest.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-atomhaltige Reste substituiert sein und/oder N-, O-, P-, S-atomhaltige Reste im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein (C₃-C₈)-Cycloalkyl-(C₁-C₈)-Alkylrest bezeichnet einen wie oben dargestellten Cycloalkylrest, welcher über einen wie oben angegebenen Alkylrest an das Molekül gebunden ist.

(C₁-C₁₈)-Acyloxy bedeutet im Rahmen der Erfindung einen wie oben definierten Alkylrest mit max. 18 C-Atomen, welcher über eine COO-Funktion an das Molekül gebunden ist. Für (C₁-C₈)-Acyloxy gilt entsprechendes.

(C₁-C₁₈)-Acyl bedeutet im Rahmen der Erfindung einen wie oben definierten Alkylrest mit max. 18 C-Atomen, welcher über eine CO-Funktion an das Molekül gebunden ist. Für (C₁-C₈)-Acyl gilt entsprechendes.

(C₁-C₈)-Alkyloxycarbonylrest bedeutet im Rahmen der Erfindung einen wie oben definierten Alkylrest mit max. 8 C-Atomen, welcher über eine OCO-Funktion an das Molekül gebunden ist.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste, welche ggf. mit (C₁-C₈)-Alkoxy, NR⁶R⁷, (C₁-C₈)-Acyl, (C₁-C₈)-Acyloxy substituiert sein können.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Rest angesehen, wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl.

Unter einem (C₄-C₁₉)-Heteroaralkyl wird ein dem (C₇-C₁₉)-Aralkylrest entsprechendes heteroaromatisches System verstanden.

Unter Carboxyschutzgruppe wird eine wie in J. Jones, The Chemical Synthesis of Peptides, Oxford Science Pub. 1991, S. 33ff dargestellte Gruppe verstanden.

Als Halogene kommen Fluor, Chlor, Brom und Iod in Frage.

Unter Salzen versteht man ionische Additionsverbindungen aus starken Säuren wie HCl, HBr, H₂SO₄, H₃PO₄, CF₃COOH, p-Toluolsulfonsäure, Methansulfonsäure und dem betrachteten Molekül.

PEG bedeutet Polyethylenglykol.

Unter dem Begriff enantiomerenangereichert wird im Rahmen der Erfindung der Anteil eines Enantiomers im Gemisch mit seiner optischen Antipode in einem Bereich von >50 % und <100 % verstanden.

Unter dem Begriff diastereomerenangereichert versteht man den Überschuß eines Diastereomers gegenüber einem oder mehreren anderen.

Die Nennung der erfindungsgemäßen Komplexe und Liganden beinhaltet im Rahmen der Erfindung alle möglichen Diastereomere, wobei auch die beiden optischen Antipoden eines jeweiligen Diastereomeren benannt sein sollen.

Unter Polymere werden im Rahmen der Erfindung eine polymere Matrix, wie z. B. lineares PMMA, Polystyrol oder PEG sowie nichtlineare Dendrimere, verstanden.

Die folgenden Beispiele sollen die Erfindung erläutern.

### Beispiele:

### Hydrierung von Ketoestern

In einem 25 ml Schlenkgefäß werden 0,01 mmol Ru(COD)₂X und 0,01 mmol des Ferrocenylliganden in 1 ml Aceton unter Argon gelöst. Dann werden 0,022 mmol HBr (c = 0,3 M; hergestellt aus 48%iger HBr in einer geeigneten Menge MeOH) zur Lösung hinzugegeben und 30 min bei RT gerührt. Anschließend destilliert man das Aceton ab und löst den Rückstand in 12 ml des entsprechenden Lösungsmittels. Nach Zugabe von 1 mmol des Ketoesters wird die Lösung unter Argon in einen 100 ml Stahlautoklaven überführt und nach mehrmaligem Spülen mit H₂ für 10 min bei dem entsprechenden Wasserstoffdruck auf Reaktionstemperatur erwärmt. Anschließend läßt man die Mischung 24 h rühren, filtriert und ermittelte den Enantiomerenüberschuß per HPLC.

### Trimethylbrenztraubensäureethylester:

Lösungsmittel: EtOH
Reaktionstemp.: 50°C
Druck: 3000 kPa

Der Umsatz wurde mittels ¹H-NMR bestimmt. Der Enantiomerüberschuss wurde mittels HPLC bestimmt (Chiralcel OD, n-Heptan / Isopropanol 99 :1; flow 0.6 mL / min, T = 20 °C: t_{R} = 10.55 (*S*), 12.25 (*R*).

### Benzylbrenztraubensäureethylester:

Lösungsmittel: EtOH
Reaktionstemp.: 50°C
Druck: 3000 kPa

Der Umsatz wurde mittels ¹H-NMR bestimmt. Der Enantiomerüberschuss wurde mittels HPLC bestimmt (Chiralcel OD, n-Heptan / Isopropanol 95 :5; flow 0.6 mL / min, T = 20 °C: t_{R} = 14.73 (*S*), 23.44 (*R*).

### Hydrierung von ungesättigten Estern/Säuren

In einem 25 ml Schlenkgefäß werden 0,01 mmol Ru(COD)₂X und 0,01 mmol des Ferrocenylliganden in 12 ml des entsprechenden Lösungsmittels gelöst. Nach Zugabe von 1 mmol des ungesättigten Esters wird die Lösung unter Argon in einen 100 ml Stahlautoklaven überführt und nach mehrmaligem Spülen mit H₂ für 10 min bei dem entsprechenden Wasserstoffdruck auf Reaktionstemperatur erwärmt. Anschließend läßt man die Mischung 24 h rühren, filtriert, (verestert die ggf. eingesetzte Säure mit Me₃SiCHN₂) und ermittelte den Enantiomerenüberschuß per HPLC [(Chiralcel OJ, n-Heptan / Isopropanol 95 :5; flow 0.6 mL / min, T = 20 °C: t_{R} = 18.05 (*S*), 21.13 (*R*)] bestimmt.

### α-Phenylacrylsäure:

Lösungsmittel: THF
Reaktionstemp.: 60°C
Druck: 5000 kPa

### α-Phenylacrylsäuremethylester:

Lösungsmittel: MeOH
Reaktionstemp.: 60°C
Druck 5000 kPa

## Patentansprüche

1. Verfahren zur homogenen katalytischen enantioselektiven Hydrierung von Verbindungen der allgemeinen Formel (I) 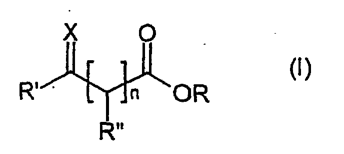 worin
n 0 ist,
R = H, (C₁-C₁₈)-Alkyl, (C₆-C₁₈)-Aryl, (C₇-C₁₉)-Aralkyl, (C₁-C₈)-Alkyl-(C₆-C₁₈)-Aryl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
oder eine Carboxyschutzgruppe bedeutet,
R' = H, (C₁-C₁₈)-Alkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, wobei die eben genannten Reste mit (C₁-C₈)-Acyl oder (C₁-C₈)-Alkyloxycarbonyl substituiert sein können, bedeutet,
wobei (C₃-C₁₈)-Heteroarylrest ein fünf-, sechs- oder siebengliedriges aromatisches Ringsystem aus 3 bis 18 C-Atomen, welches Heteroatome ausgewählt aus Stickstoff, Sauerstoff oder Schwefel im Ring aufweist bezeichnet,
X = O, CHR'', NR'', ist,
R" = H, OH, R', (C₁-C₁₈)-Alkoxy, (C₂-C₁₈)-Alkoxyalkyl, (C₁-C₁₈)-Acyl, (C₁-C₁₈)-Acyloxy sein kann, wobei für verschiedene Positionen R'' verschiedene Gestalt annehmen kann,
oder R und R' oder R' und R'' oder R'' und R sind über eine (C₁-C₄)-Brücke miteinander verbunden, welche einfach oder mehrfach mit linear oder verzweigten (C₁-C₈)-Alkyl, (C₁-C₈)-Acyl, (C₁-C₈)-Alkoxy, (C₂-C₈)-Alkoxyalkylresten substituiert sein und/oder im Ring Heteroatome ausgewählt aus N, O, P, S enthalten kann,
oder worin
n 1 ist,
R = H, (C₁-C₁₈)-Alkyl, (C₆-C₁₈)-Aryl, (C₇-C₁₉)-Aralkyl, (C₁-C₈)-Alkyl-(C₆-C₁₈)-Aryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
oder eine Carboxyschutzgruppe bedeutet,
R' = H, (C₁-C₁₈)-Alkyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl, wobei die eben genannten Reste mit (C₁-C₈)-Acyl oder (C₁-C₈)-Alkyloxycarbonyl substituiert sein können, bedeutet,
wobei (C₃-C₁₈)-Heteroarylrest ein fünf-, sechs- oder siebengliedriges aromatisches Ringsystem aus 3 bis 18 C-Atomen, welches Heteroatome ausgewählt aus Stickstoff, Sauerstoff oder Schwefel im Ring aufweist bezeichnet,
X = NR'', ist,
R'' = H, OH, R', (C₁-C₁₈)-Alkoxy, (C₂-C₁₈)-Alkoxyalkyl, (C₁-C₁₈)-Acyl, (C₁-C₁₈)-Acyloxy sein kann, wobei für verschiedene Positionen R'' verschiedene Gestalt annehmen kann,
mit Hilfe von Katalysatoren der allgemeinen Formel (II) 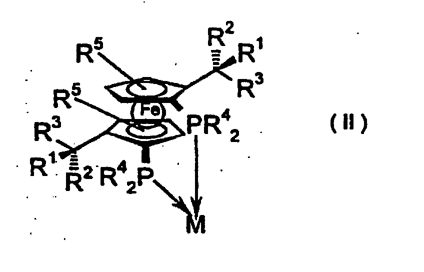 worin
R¹, R² unabhängig voneinander bedeuten H, O(C₁-C₈)-Acyl, N(C₁-C₈)-Alkyl₂, (C₁-C₈)-Alkyl
R³ bedeutet (C₆-C₁₈)-Aryl,
R⁴ bedeutet Phenyl,
R⁵ bedeutet H.
oder R und R' oder R' und R'' oder R'' und R sind über eine (C₁-C₄)-Brücke miteinander verbunden, welche einfach oder mehrfach mit linear oder verzweigten (C₁-C₈)-Alkyl, (C₁-C₈)-Acyl, (C₁-C₈)-Alkoxy, (C₂-C₈)-Alkoxyalkylresten substituiert sein und/oder im Ring Heteroatome ausgewählt aus N, O, P, S enthalten kann, und M ein Metallatom oder -ion der Nebengruppe 7 oder 8, wie z. B. Co, Ni, Rh, Ru, Ir, Pd, Re oder Pt ist.

2. Verfahren nach Anspruch 1,
**dadurch gekennzeichnet, daß**
die Temperatur bei der Umsetzung zwischen 0°C und 150°C, vorzugsweise zwischen 20°C und 80°C, liegt.

3. Verfahren nach einem oder mehreren der vorhergehenden Ansprüche,
**dadurch gekennzeichnet, daß**
der Wasserstoffdruck während der Reaktion zwischen 10 kPa und 10000 kPa, vorzugsweise zwischen 50 kPa und 8000 kPa, liegt.

4. Verfahren nach einem oder mehreren der vorhergehenden Ansprüche,
**dadurch gekennzeichnet, daß**
man als Lösungsmittel bei der Reaktion Ether, wie THF, DME, MTBE, Alkohole, wie MeOH, EtOH, Propanol, Butanol, verwendet.

## Claims

1. Process for the homogeneous, catalytic, enantioselective hydrogenation of compounds of the general formula (I) 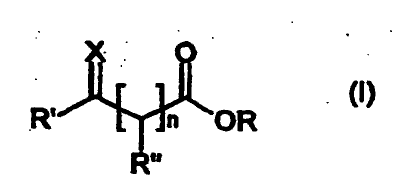 wherein
n is 0, 1,
R = H, (C₁-C₁₈)-alkyl, (C₆-C₁₈)-aryl, (C₇-C₁₉)-aralkyl, (C₁-C₈)-alkyl- (C₆-C₁₈)-aryl, (C₃-C₈)-cycloalkyl, (C₁-C₈)-alkyl- (C₃-C₈)-cycloalkyl, (C₃-C₈)-cycloalkyl- (C₁-C₈)-alkyl or a carboxy protective group,
R' = H, (C₁-C₁₈)-alkyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl- (C₆-C₁₈)-aryl, (C₁-C₈)-alkyl- (C₃-C₁₉)-heteroaryl, (C₃-C₈)-cycloalkyl, (C₁-C₈)-alkyl- (C₃-C₈)-cycloalkyl, (C₃-C₈)-cycloalkyl-(C₁-C₈)-alkyl, said residues being optionally substituted with (C₁-C₈)-acyl or (C₁-C₈)-alkoxycarbonyl, wherein (C₃-C₁₈)-heteroaryl residue denotes a five-, six- or seven-membered aromatic ring system of 3 to 18 C atoms containing heteroatoms selected from nitrogen, oxygen or sulfur in the ring,
X = O, CHR", NR",
R" can be H, OH, R', (C₁-C₁₈)-alkoxy, (C₂-C₁₈)-alkoxyalkyl, (C₁-C₁₈)-acyl, (C₁-C₁₈)-acyloxy, with R" being able to take a different form for different positions,
or R and R' or R' and R" or R" and R are connected together via a (C₁-C₄) bridge, which can be mono- or polysubstituted with linear or branched (C₁-C₈)-alkyl, (C₁-C₈)-acyl, (C₁-C₈)-alkoxy, (C₂-C₈)-alkoxyalkyl residues and/or can contain heteroatoms in the ring, selected from N, O, P, S,
or
wherein
n is 1,
R = H, (C₁-C₁₈)-alkyl, (C₆-C₁₈)-aryl, (C₇-C₁₉)-aralkyl, (C₁-C₈)-alkyl-(C₆-C₁₈)-aryl, (C₃-C₈)-cycloalkyl, (C₁-C₈)-alkyl- (C₃-C₈)-cycloalkyl, (C₃-C₈)-cycloalkyl- (C₁-C₈)-alkyl or a carboxy protective group,
R' = H, (C₁-C₁₈)-alkyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl- (C₆-C₁₈)-aryl, (C₁-C₈)-alkyl-(C₃-C₁₉)-heteroaryl, (C₃-C₈)-cycloalkyl, (C₁-C₈)-alkyl-(C₃-C₈)-cycloalkyl, (C₃-C₈)-cycloalkyl-(C₁-C₈)-alkyl, said residues being optionally substituted with (C₁-C₈)-acyl or (C₁-C₈)-alkoxycarbonyl, wherein (C₃-C₁₈)-heteroaryl residue denotes a five-, six- or seven-membered aromatic ring system of 3 to 18 C atoms containing heteroatoms selected from nitrogen, oxygen or sulfur in the ring,
X = NR",
R" can be H, OH, R', (C₁-C₁₈)-alkoxy, (C₂-C₁₈)-alkoxyalkyl, (C₁-C₁₈)-acyl, (C₁-C₁₈)-acyloxy, with R" being able to take a different form for different positions,
or R and R' or R' and R" or R" and R are connected together via a (C₁-C₄) bridge, which can be mono- or polysubstituted with linear or branched (C₁-C₈)-alkyl, (C₁-C₈)-acyl, (C₁-C₈)-alkoxy, (C₂-C₈)-alkoxyalkyl residues and/or can contain heteroatoms in the ring, selected from N, O, P, S,
with the aid of catalysts of the general formula (II) 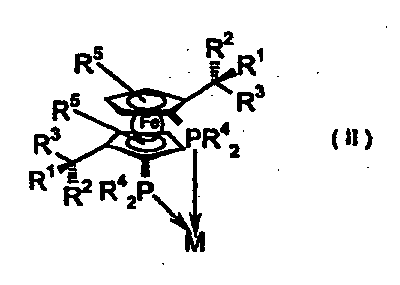 wherein
R¹, R², independently of one another, denote H, O(C₁-C₈)-acyl, N(C₁-C₈)-alkyl₂, (C₁-C₈)-alkyl,
R³ denotes (C₆-C₁₈)-aryl,
R⁴ denotes phenyl,
R⁵ denotes H,
and M is a metal atom or ion from subgroup 7 or 8, such as e.g. Co, Ni, Rh, Ru, Ir, Pd, Re or Pt.

2. Process according to claim 1, **characterised in that** the temperature during the reaction is between 0°C and 150°C, preferably between 20°C and 80°C.

3. Process according to one or more of the above claims, **characterised in that** the hydrogen pressure during the reaction is between 10 kPa and 10000 kPa, preferably between 50 kPa and 8000 kPa.

4. Process according to one or more of the above claims, **characterised in that** ethers, such as THF, DME, MTBE, or alcohols, such as MeOH, EtOH, propanol or butanol, are used as solvents during the reaction.

## Revendications

1. Procédé d'hydrogénation énantiosélective homogène catalytique de composés de formule générale (I) 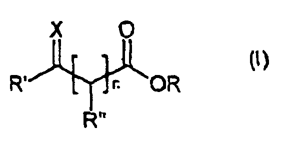 dans laquelle
n représente 0, 1
R = H, alkyle en C₁ à C₁₈, aryle en C₆ à C₁₈, aralkyle en C₇ à C₁₉, (alkyle en C₁ à C₈)-(aryle en C₆ à C₁₈), cycloalkyle en C₃ à C₈, (alkyle en C₁ à C₈)-(cycloalkyle en C₃ à C₈), (cycloalkyle en C₃ à C₈)-(alkyle en C₁ à C₈) ou un groupe de protection de la fonction carboxy,
R' = H, alkyle en C₁ à C₁₈, aryle en C₆ à C₁₈, aralkyle en C₇ à C₁₉, hétéroaryle en C₃ à C₁₈, hétéroalkyle en C₄ à C₁₉, (alkyle en C₁ à C₈)-(aryle en C₆ à C₁₈), (alkyle en C₁ à C₈)-(hétéroaryle en C₃ à C₁₉), cycloalkyle en C₃ à C₈, (alkyle en C₁ à C₈)-(cycloalkyle en C₃ à C₈), (cycloalkyle en C₃ à C₈)-(alkyle en C₁ à C₈), où les radicaux susmentionnés peuvent être substitués par acyle en C₁ à C₈ ou (alkyle en C₁ à C₈)oxycarbonyle, le radical hétéroaryle en C₃ à C₁₈ signifiant un système cyclique à cinq, six ou sept chaînons comprenant 3 à 18 atomes de carbone, présentant des hétéroatomes choisis parmi l'azote, l'oxygène ou le soufre dans le cycle
X = O, CHR", NR",
R" = H, OH, R', alcoxy en C₁ à C₁₈, alcoxyalkyle en C₂ à C₁₈, acyle en C₁ à C₁₈, acyloxy en C₁ à C₁₈, R" dans les différentes positions pouvant prendre différentes significations, ou R et R' ou R' et R" ou R" et R sont liés l'un à l'autre via un pont en C₁ à C₄, qui peut être monosubstitué ou polysubstitué par des radicaux alkyle en C₁ à C₈, acyle en C₁ à C₈, alcoxy en C₁ à C₈, alcoxyalkyle en C₂ à C₈ linéaires ou ramifiés et/ou contenir dans le cycle des hétéroatomes choisis parmi N, O, P, S
ou dans laquelle
n représente 1
R = H, alkyle en C₁ à C₁₈, aryle en C₆ à C₁₈, aralkyle en C₇ à C₁₉, (alkyle en C₁ à C₈)-(aryle en C₆ à C₁₈), cycloalkyle en C₃ à C₈, (alkyle en C₁ à C₈)-(cycloalkyle en C₃ à C₈), (cycloalkyle en C₃ à C₈)-(alkyle en C₁ à C₈) ou un groupe de protection de la fonction carboxy,
R' = H, alkyle en C₁ à C₁₈, aryle en C₆ à C₁₈, aralkyle en C₇ à C₁₉, hétéroaryle en C₃ à C₁₈, hétéroalkyle en C₄ à C₁₉, (alkyle en C₁ à C₈)-(aryle en C₆ à C₁₈), (alkyle en C₁ à C₈)-(hétéroaryle en C₃ à C₁₉), cycloalkyle en C₃ à C₈, (alkyle en C₁ à C₈)-(cycloalkyle en C₃ à C₈), (cycloalkyle en C₃ à C₈)-(alkyle en C₁ à C₈), où les radicaux susmentionnés peuvent être substitués par acyle en C₁ à C₈ ou (alkyle en C₁ à C₈)oxycarbonyle, le radical hétéroaryle en C₃ à C₁₈ signifiant un système cyclique à cinq, six ou sept chaînons comprenant 3 à 18 atomes de carbone, présentant des hétéroatomes choisis parmi l'azote, l'oxygène ou le soufre dans le cycle
X = NR",
R" = H, OH, R', alcoxy en C₁ à C₁₈, alcoxyalkyle en C₂ à C₁₈, alcoxyalkyle en C₂ à C₁₈, acyle en C₁ à C₁₈, acyloxy en C₁ à C₁₈, R" dans les différentes positions pouvant prendre différentes significations,
ou R et R' ou R' et R" ou R" et R sont liés l'un à l'autre via un pont en C₁ à C₄, qui peut être monosubstitué ou polysubstitué par des radicaux alkyle en C₁ à C₈, acyle en C₁ à C₈, alcoxy en C₁ à C₈, alcoxyalkyle en C₂ à C₈ linéaires ou ramifiés et/ou contenir dans le cycle des hétéroatomes choisis parmi N, O, P, S
à l'aide de catalyseurs de formule générale (II) 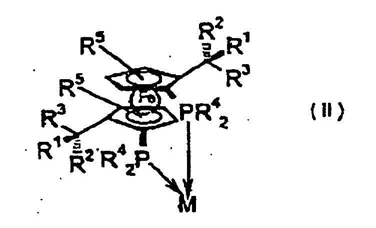 dans laquelle R¹ et R² représentent indépendamment l'un de l'autre H, O (acyle en C₁ à C₃), N(alkyle en C₁ à C₈)₂, alkyle en C₁ à C₈,
R³ signifie aryle en C₆ à C₁₈,
R⁴ signifie phényle,
R⁵ signifie H
et M représente un atome ou un ion métallique du 7ème ou 8ème groupe secondaire, tel que par exemple Co, Ni, Rh, Ru, Ir, Pd, Re ou Pt.

2. Procédé selon la revendication 1, **caractérisé en ce que** la température lors de la transformation est située entre 0°C et 150°C, de préférence entre 20°C et 80°C.

3. Procédé selon l'une ou plusieurs des revendications précédentes, **caractérisé en ce que** la pression d'hydrogène pendant la réaction est située entre 10 kPa et 10000 kPa, de préférence entre 50 kPa et 8000 kPa.

4. Procédé selon l'une ou plusieurs des revendications précédentes, **caractérisé en ce qu'**on utilise comme solvant lors de la réaction des éthers tels que le THF, le DME, le MTBE, des alcools tels que le MeOH, l'EtOH, le propanol, le butanol.
